# EUROPEAN PATENT APPLICATION

(11) **EP 3 909 620 A1**
(43) Date of publication of application: **17.11.2021**
(21) Application number: 20208108.9
(22) Date of filing: 17.11.2020
(51) Int. Cl.: A61M 1/16

(54) **HEMODIALYZER AND HEMODIALYSIS SYSTEM**

(30) Priority: 11.05.2020 US 202016872311
(71) Applicant: QISDA Corporation, Taoyuan City 333 (TW)
(72) Inventor: Yang, Chin-Wen, 220 New Taipei City (TW); Huang, Shih-Hsiu, 324 Taoyuan City (TW); Lin, Shu-Chuen, 114 Taipei City (TW); Shih, Chien-Hua, 105 Taipei City (TW)
(74) Representative: Straus, Alexander

(57) **Abstract**

A hemodialyzer (10) includes a housing (100), a hollow fiber membrane (102), a first cap (104), a second cap (106) and a hydrophilic material (114). The hollow fiber membrane (102) is disposed in the housing (100). The first cap (104) is disposed on a blood inlet end (1000) of the housing (100) and the second cap (106) is disposed on a blood outlet end (1002) of the housing (100), wherein the blood inlet end (1000) is opposite to the blood outlet end (1002). The hydrophilic material (114) is formed on an inner surface of at least one of the first cap (104) and the second cap (106).

## Description

### Field of the Invention

The present invention relates to a hemodialyzer, particularly a hemodialyzer capable of avoiding blood coagulation and a hemodialysis system equipped with the hemodialyzer.

### Background of the Invention

At present, a hemodialyzer is widely used for blood purification in patients suffering from renal insufficiency. The hemodialyzer includes a housing, a hollow fiber membrane and two caps, wherein the hollow fiber membrane is disposed in the housing and the two caps are disposed on opposite ends of the housing. When the hemodialyzer is used for hemodialysis, the blood flows into the housing through the cap disposed on a blood inlet end, passes through the hollow fiber membrane, and then flows out of the housing through the other cap disposed on a blood outlet end. In general, the caps are made of an engineering plastic, such as polycarbonate (PC), polypropylene (PP), and so on. Some substances including cells and proteins in the blood will be adsorbed on an inner surface of the cap during hemodialysis, such that blood coagulation usually occurs on the inner surface of the cap. Consequently, the safety of hemodialysis is affected.

### Summary of the Invention

The present invention aims at providing a hemodialyzer capable of avoiding blood coagulation and a hemodialysis system equipped with the hemodialyzer, thereby resolving the aforesaid problems.

This is achieved by a hemodialyzer according to claim 1 and a hemodialysis system according to claim 5. The dependent claims pertain to corresponding further developments and improvements.

As will be seen more clearly from the detailed description following below, the claimed hemodialyzer includes a housing, a hollow fiber membrane, a first cap, a second cap and a hydrophilic material. The hollow fiber membrane is disposed in the housing. The first cap is disposed on a blood inlet end of the housing and the second cap is disposed on a blood outlet end of the housing, wherein the blood inlet end is opposite to the blood outlet end. The hydrophilic material is formed on an inner surface of at least one of the first cap and the second cap.

As will be seen more clearly from the detailed description following below, the claimed hemodialysis system includes a hemodialyzer, a first blood tube, a second blood tube, a first dialysate tube, a second dialysate tube and a hydrophilic material. The hemodialyzer includes a housing, a hollow fiber membrane, a first cap and a second cap. The hollow fiber membrane is disposed in the housing. The first cap is disposed on a blood inlet end of the housing and the second cap is disposed on a blood outlet end of the housing, wherein the blood inlet end is opposite to the blood outlet end. The housing has a dialysate inlet port and a dialysate outlet port. The first blood tube is connected to the first cap. The second blood tube is connected to the second cap. The first dialysate tube is connected to the dialysate inlet port. The second dialysate tube is connected to the dialysate outlet port. The hydrophilic material is formed on an inner surface of at least one of the first cap and the second cap and formed on an inner surface of at least one of the first blood tube and the second blood tube.

### Brief Description of the Drawings

In the following, the invention is further illustrated by way of example, taking reference to the accompanying drawings thereof:
FIG. 1 is a perspective view illustrating a hemodialyzer according to an embodiment of the invention,
FIG. 2 is a sectional view illustrating the hemodialyzer shown in FIG. 1,
FIG. 3 is a partial exploded view illustrating the hemodialyzer shown in FIG. 1, and
FIG. 4 is a schematic view illustrating a hemodialysis system equipped with the hemodialyzer shown in FIG. 3.

### Detailed Description

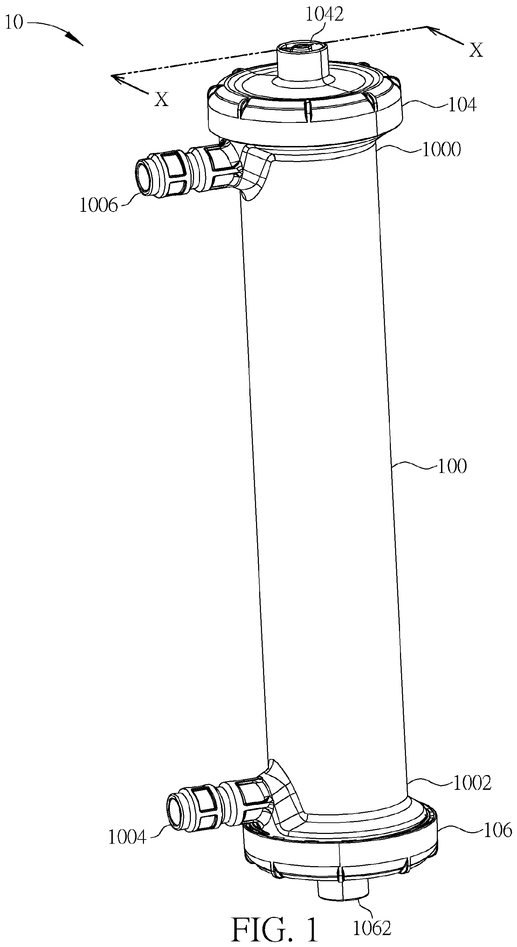

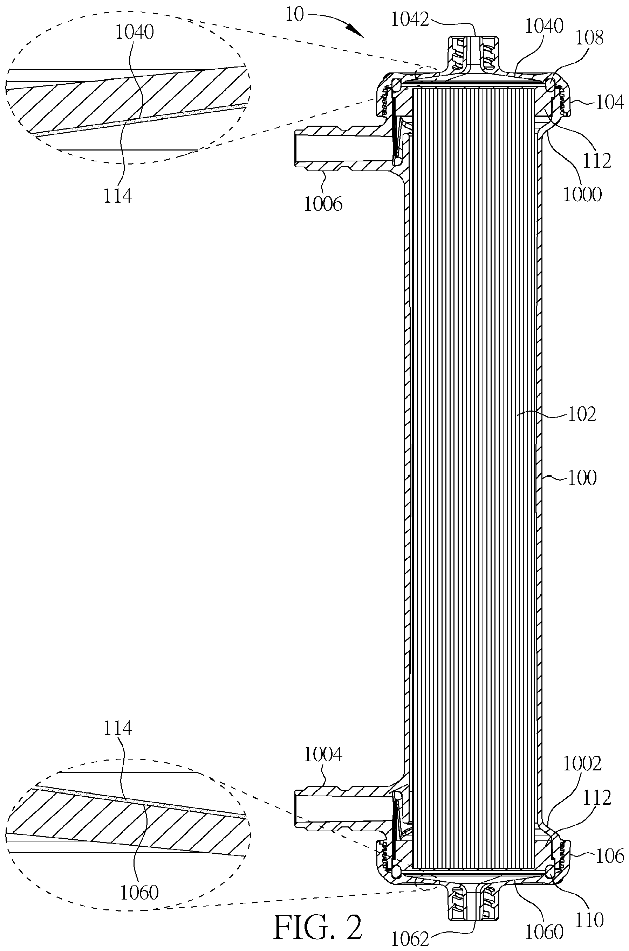

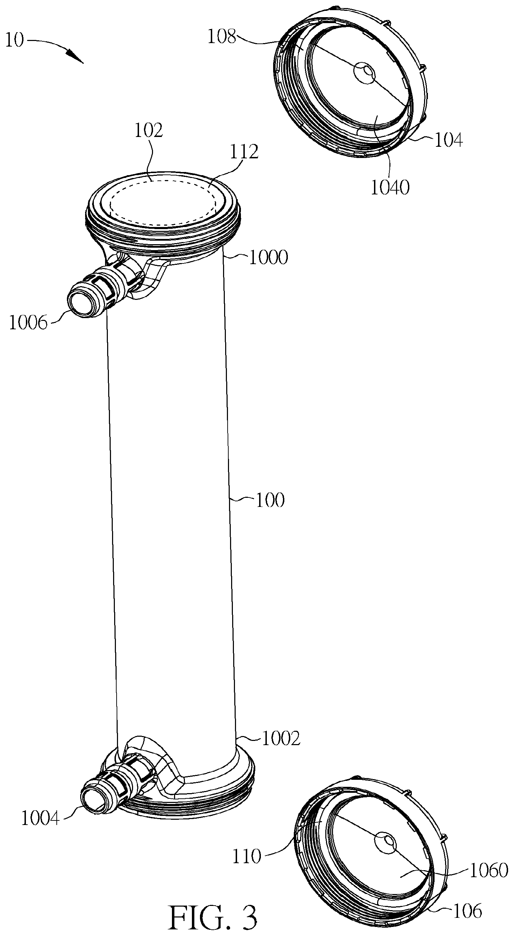

Referring to FIGs. 1 to 3, FIG. 1 is a perspective view illustrating a hemodialyzer 10 according to an embodiment of the invention, FIG. 2 is a sectional view illustrating the hemodialyzer 10 along lien X-X shown in FIG. 1, and FIG. 3 is a partial exploded view illustrating the hemodialyzer 10 shown in FIG. 1.

As shown in FIGs. 1 to 3, the hemodialyzer 10 includes a housing 100, a hollow fiber membrane 102, a first cap 104, a second cap 106, a first sealing ring 108 and a second sealing ring 110. The hollow fiber membrane 102 is disposed in the housing 100. In practical applications, an adhesive 112 (e.g. polyurethane (PU) adhesive) may be used to fix the hollow fiber membrane 102 in the housing 100. The first cap 104 is disposed on a blood inlet end 1000 of the housing 100 and the second cap 106 is disposed on a blood outlet end 1002 of the housing 100, wherein the blood inlet end 1000 is opposite to the blood outlet end 1002.

The first sealing ring 108 is disposed on an inner surface 1040 of the first cap 104 and the second sealing ring 110 is disposed on an inner surface 1060 of the second cap 106. In this embodiment, the first sealing ring 108 and the second sealing ring 110 may be, but not limited to, O-rings. Furthermore, the first cap 104 has a blood inlet port 1042 and the second cap 106 has a blood outlet port 1062, wherein the blood inlet port 1042 and the blood outlet port 1062 are configured to connect blood tubes, so as to form a blood loop. Still further, the housing 100 has a dialysate inlet port 1004 and a dialysate outlet port 1006, wherein the dialysate inlet port 1004 and the dialysate outlet port 1006 are configured to connect dialysate tubes, so as to form a dialysate loop.

When the hemodialyzer 10 is used for hemodialysis, the blood flows into the housing 100 through the blood inlet port 1042 of the first cap 104, passes through the hollow fiber membrane 102, and then flows out of the housing 100 through the blood outlet port 1062 of the second cap 106. The hollow fiber membrane 102 dialyze the blood by tangential flow filtration. Furthermore, the dialysate flows into the housing 100 through the dialysate inlet port 1004 and then flows out of the housing 100 through the dialysate outlet port 1006. It should be noted that the principle of hemodialysis is well known by one skilled in the art, so the explanation will not be depicted herein.

In this embodiment, the hemodialyzer 10 further includes a hydrophilic material 114 formed on the inner surface 1040, 1060 of at least one of the first cap 104 and the second cap 106. When the hydrophilic material 114 is formed on the inner surfaces 1040, 1060 of the first cap 104 and the second cap 106 (as shown in FIG. 2), some substances including cells and proteins in the blood will not be adsorbed on the inner surfaces 1040, 1060 of the first cap 104 and the second cap 106. Accordingly, the invention can avoid blood coagulation formed on the first cap 104 and the second cap 106 during hemodialysis, so as to ensure the safety of hemodialysis. In this embodiment, the hydrophilic material 114 may be, but not limited to, phosphorylcholine.

In an embodiment, the invention may dissolve the hydrophilic material 114 in a solution and then soak the first cap 104 and the second cap 106 in the solution. Then, the first cap 104 and the second cap 106 are taken out of the solution and cured with high temperature, so as to solidify the hydrophilic material 114 on the first cap 104 and the second cap 106.

In another embodiment, the invention may coat hydrophilic material 114 on the inner surfaces 1040, 1060 of the first cap 104 and the second cap 106. Then, the inner surfaces 1040, 1060 of the first cap 104 and the second cap 106 are irradiated by ultraviolet (UV) rays, so as to solidify the hydrophilic material 114 on the first cap 104 and the second cap 106.

In general, blood coagulation is easier to be formed on the first cap 104 disposed on the blood inlet end 1000. Accordingly, in another embodiment, the invention may only form the hydrophilic material 114 on the inner surface 1040 of the first cap 104. Needless to say, the invention may only form the hydrophilic material 114 on the inner surface 1060 of the second cap 106 according to practical applications.

In another embodiment, the invention may further form the hydrophilic material 114 on an inner surface of at least one of the blood inlet port 1042 and the blood outlet port 1062, so as to avoid blood coagulation formed on the blood inlet port 1042 and/or the blood outlet port 1062 during hemodialysis. It should be noted that the invention may form the hydrophilic material 114 on the inner surface of at least one of the blood inlet port 1042 and the blood outlet port 1062 according to the aforesaid manners, so the repeated explanation will not be depicted herein again.

In another embodiment, the invention may further form the hydrophilic material 114 on at least one of the first sealing ring 108 and the second sealing ring 110, so as to avoid blood coagulation formed on the first sealing ring 108 and/or the second sealing ring 110 during hemodialysis. It should be noted that the invention may form the hydrophilic material 114 on at least one of the first sealing ring 108 and the second sealing ring 110 according to the aforesaid manners, so the repeated explanation will not be depicted herein again.

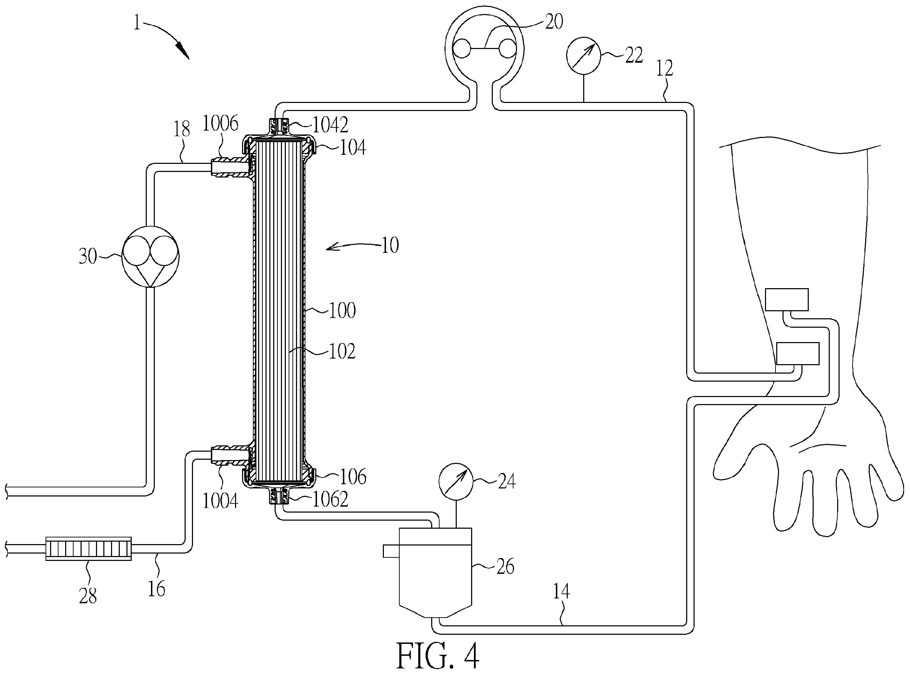

Referring to FIG. 4, FIG. 4 is a schematic view illustrating a hemodialysis system 1 equipped with the hemodialyzer 10 shown in FIG. 3. As shown in FIG. 4, the hemodialysis system 1 includes a hemodialyzer 10, a first blood tube 12, a second blood tube 14, a first dialysate tube 16, a second dialysate tube 18, a blood pump 20, an arterial pressure monitor 22, a venous pressure monitor 24, a bubble detector 26, a heater 28 and a dialysate pump 30. The structure and principle of the hemodialyzer 10 are mentioned in the above and those will not be depicted herein again.

The first blood tube 12 is connected to the blood inlet port 1042 of the first cap 104 and the second blood tube 14 is connected to the blood outlet port 1062 of the second cap 106, so as to form a blood loop. The first dialysate tube 16 is connected to the dialysate inlet port 1004 of the housing 100 and the second dialysate tube 18 is connected to the dialysate outlet port 1006 of the housing 100, so as to form a dialysate loop. The blood pump 20 and the arterial pressure monitor 22 are connected to the first blood tube 12. The venous pressure monitor 24 and the bubble detector 26 are connected to the second blood tube 14. The heater 28 is connected to the first dialysate tube 16. The dialysate pump 30 is connected to the second dialysate tube 18. It should be noted that the principles of the blood pump 20, the arterial pressure monitor 22, the venous pressure monitor 24, the bubble detector 26, the heater 28 and the dialysate pump 30 are well known by one skilled in the art, so the explanation will not be depicted herein.

In this embodiment, the invention may further form the hydrophilic material 114 on an inner surface of at least one of the first blood tube 12 and the second blood tube 14, so as to avoid blood coagulation formed on the first blood tube 12 and/or the second blood tube 14 during hemodialysis.

As mentioned in the above, the invention forms the hydrophilic material on the inner surface of the cap, such that some substances including cells and proteins in the blood will not be adsorbed on the inner surface of the cap. Furthermore, the invention may further form the hydrophilic material on the inner surface of the blood tube connected to the cap, such that some substances including cells and proteins in the blood will not be adsorbed on the inner surface of the blood tube. Still further, the invention may further form the hydrophilic material on the inner surface of the blood inlet port and/or the blood outlet port, such that some substances including cells and proteins in the blood will not be adsorbed on the inner surface of the blood inlet port and/or the blood outlet port. Moreover, the invention may further form the hydrophilic material on the sealing ring, such that some substances including cells and proteins in the blood will not be adsorbed on the sealing ring. Accordingly, the invention can avoid blood coagulation during hemodialysis, so as to ensure the safety of hemodialysis.

## Claims

1. A hemodialyzer (10) comprising:
a housing (100);
a hollow fiber membrane (102) disposed in the housing (100);
a first cap (104) disposed on a blood inlet end (1000) of the housing (100);
a second cap (106) disposed on a blood outlet end (1002) of the housing (100), the blood inlet end (1000) being opposite to the blood outlet end (1002); and
**characterized by**:
a hydrophilic material (114) formed on an inner surface of at least one of the first cap (104) and the second cap (106).

2. The hemodialyzer (10) of claim 1 further **characterized in that** the first cap (104) has a blood inlet port (1042), the second cap (106) has a blood outlet port (1062), and the hydrophilic material (114) is further formed on an inner surface of at least one of the blood inlet port (1042) and the blood outlet port (1062).

3. The hemodialyzer (10) of claim 1 further **characterized in that** the hemodialyzer (10) comprises a first sealing ring (108) and a second sealing ring (110), the first sealing ring (108) is disposed on the inner surface of the first cap (104), the second sealing ring (110) is disposed on the inner surface of the second cap (106), and the hydrophilic material (114) is further formed on at least one of the first sealing ring (108) and the second sealing ring (110).

4. The hemodialyzer (10) of claim 1 further **characterized in that** the hydrophilic material (114) is phosphorylcholine.

5. A hemodialysis system (1) comprising:
a hemodialyzer (10) comprising a housing (100), a hollow fiber membrane (102), a first cap (104) and a second cap (106), the hollow fiber membrane (102) being disposed in the housing (100), the first cap (104) being disposed on a blood inlet end (1000) of the housing (100), the second cap (106) being disposed on a blood outlet end (1002) of the housing (100), the blood inlet end (1000) being opposite to the blood outlet end (1002), the housing (100) having a dialysate inlet port (1004) and a dialysate outlet port (1006);
a first blood tube (12) connected to the first cap (104);
a second blood tube (14) connected to the second cap (106);
a first dialysate tube (16) connected to the dialysate inlet port (1004);
a second dialysate tube (18) connected to the dialysate outlet port (1006); and
**characterized by**:
a hydrophilic material (114) formed on an inner surface of at least one of the first cap (104) and the second cap (106) and formed on an inner surface of at least one of the first blood tube (12) and the second blood tube (14).

6. The hemodialysis system (1) of claim 5 further **characterized in that** the first cap (104) has a blood inlet port (1042), the first blood tube (12) is connected to the blood inlet port (1042), the second cap (106) has a blood outlet port (1062), the second blood tube (14) is connected to the blood outlet port (1062), and the hydrophilic material (114) is further formed on an inner surface of at least one of the blood inlet port (1042) and the blood outlet port (1062).

7. The hemodialysis system (1) of claim 5 further **characterized in that** the hemodialyzer (10) further comprises a first sealing ring (108) and a second sealing ring (110), the first sealing ring (108) is disposed on the inner surface of the first cap (104), the second sealing ring (110) is disposed on the inner surface of the second cap (106), and the hydrophilic material (114) is further formed on at least one of the first sealing ring (108) and the second sealing ring (110).

8. The hemodialysis system (1) of claim 5 further **characterized in that** the hydrophilic material (114) is phosphorylcholine.

9. The hemodialysis system (1) of claim 5 further **characterized in that** the hemodialysis system (1) further comprises:
a blood pump (20) connected to the first blood tube (12);
an arterial pressure monitor (22) connected to the first blood tube (12);
a venous pressure monitor (24) connected to the second blood tube (14);
a bubble detector (26) connected to the second blood tube (14);
a heater (28) connected to the first dialysate tube (16); and
a dialysate pump (30) connected to the second dialysate tube (18).
